# DEMANDE DE BREVET EUROPEEN

(11) **EP 0 943 347 A1**
(43) Date de publication de la demande: **22.09.1999**
(21) Numéro de dépôt: 99400666.6
(22) Date de dépôt: 18.03.1999
(51) Int. Cl.: A61L 29/00, A61L 33/00, A61L 27/00, A61M 5/315

(54) **Matériau à usage médical présentant un faible coefficient de glissement et procédé d'obtention**

(30) Priorité: 20.03.1998 FR 9803479
(71) Demandeur: Association pour les transferts de technologie Du Mans, 72000 Le Mans (FR)
(72) Inventeur: Porcheron, Christelle, 72500 Chenu (FR); Legeay, Gilbert, 72650 Saint-Saturnin (FR)
(74) Mandataire: Phélip, Bruno

(57) **Abrégé**

Matériau à faible coefficient de glissement caractérisé en ce qu'il est constitué d'un support, modifié en surface par création de sites polaires, recouvert par une couche d'au moins un polymère hydrosoluble et biocompatible.

Des instruments à usage médical, et en particulier des tubulures, constitués de ce matériau présentent l'avantage de glisser sans endommager les tissus.

## Description

La présente invention a pour objet des matériaux à usage médical présentant un faible coefficient de glissement.

Elle est en outre relative à un procédé d'obtention de ces matériaux.

L'utilisation de tubulures en milieu médical est très répandue. L'introduction de celles-ci dans le corps humain (pour des usages cardio-vasculaire, urinaire, stomacal ou autres) se fait toujours dans un conduit naturel fortement humide. La rigidité du matériau et la rugosité font que le glissement est parfois difficile. Il est nécessaire d'appliquer des forces parfois importantes pouvant entraîner des endommagements de tissus.

Ce problème se retrouve lors de l'utilisation d'autres instruments à usage médical, qui sont mis en contact avec divers tissus du corps humain ou animal.

Divers matériaux sont connus pour leur biocompatibilité, ou pour leur thrombogénicité réduite. Ainsi, la demande EP-0 611 792 décrit des matériaux de nature polymérique rendus biocompatibles par irradiation de leur surface en présence d'oxygène et greffage du monomère acrylamide.

On connaît aussi des procédés de traitement par plasma, destinés à augmenter la biocompatibilité de polymères. Ainsi, le brevet FR 2 620 624 décrit une méthode de traitement d'articles en matériaux polymériques pour présenter une hémocompatibilité améliorée et une thrombogénéicité diminuée. Le traitement est effectué dans un plasma de molécules fluorées non polymérisables et conduit au remplacement d'atomes d'hydrogène en surface par des atomes de fluor, ou des groupements en contenant.

Néanmoins, ces procédés ne sont pas destinés à diminuer le coefficient de glissement de la surface des matériaux.

La demande EP-0 611 576 décrit un procédé pour diminuer le coefficient de glissement à la surface d'instruments médicaux constitués d'une matrice contenant des groupes donneurs de protons, tels que des groupes amine. Ledit matériau est recouvert, par immersion, d'un polymère susceptible de gonfler contenant un groupe fonctionnel réagissant avec le groupe donneur de protons du matériau, puis est chauffé à une température supérieure à 100°C.

Ce type de technique présente un certain nombre d'inconvénients.

L'inconvénient principal réside néanmoins dans l'homologation de ces polymères pour leurs utilisations médicales. En effet, la synthèse de ces polymères est effectuée à partir de monomères, qui, dans leur grande majorité sont toxiques. En outre, ces polymérisations nécessitent l'utilisation de solvants qui sont tout autant toxiques.

Il est donc obligatoire de démontrer, pour chaque lot de polymères, c'est-à-dire après chaque polymérisation, que les polymères obtenus sont dépourvus de toxicité. Ces vérifications sont longues et coûteuses, et sont donc incompatibles avec une production de masse et normalisée de dispositifs médicaux d'usages courants, tels que des tubulures.

De plus, l'utilisation de solvants nécessite des installations industrielles particulières, en raison des risques généraux entraînés par l'utilisation de tels produits, en particulier vis-à-vis de l'environnement.

En outre, les procédés de synthèse de ces polymères sont compliqués et longs.

On connaît aussi un procédé consistant à recouvrir la surface à traiter d'un polymère hybride constitué de polyvinylpyrrolidone et d'esters de cellulose hydrophobes. Néanmoins, ce procédé nécessite l'utilisation de solvants organiques qui, comme indiqué ci-dessus, sont difficilement compatibles avec les exigences liées à l'homologation.

Le traitement par plasma de tubulures a aussi été envisagé. Néanmoins, un tel traitement est insuffisant en ce qu'il ne permet pas d'obtenir un coefficient de glissement suffisamment faible.

On notera en outre que les procédés de recouvrement décrits dans l'état de la technique sont décrits pour des matériaux et des polymères spécifiques et ne sont pas généralisables à tous les types de surface et de polymères.

La demanderesse s'est donc attachée à trouver un procédé de recouvrement d'instruments à usage médical, dépourvu de toute toxicité, permettant l'obtention d'un faible coefficient de glissement, et utilisable pour recouvrir divers matériaux.

Elle a trouvé que le traitement de la surface de tels instruments puis leur recouvrement par des polymères particuliers, permettait de résoudre l'ensemble de ces problèmes.

La présente invention a donc pour objet un matériau à faible coefficient de glissement, caractérisé en ce qu'il est constitué d'un support, modifié en surface par création de sites polaires, recouvert par une couche d'au moins un polymère hydrosoluble et biocompatible.

Avantageusement, le support est modifié en surface sur une épaisseur comprise entre environ 10 et 500 nm, préférentiellement entre 30 et 100 nm. La création de sites polaires correspond principalement à l'augmentation de la proportion de groupements carbonyle, hydroxy ou amine, et des radicaux libres. Les radicaux libres se recombinent entre eux, ou avec l'oxygène de l'air, créant ainsi des sites polaires.

Avantageusement, ledit polymère est un dérivé cellulosique, une polyvinylpyrrolidone, ou un copolymère de polyvinylpyrrolidone, un polypropylèneglycol, un polyacrylamide, un polyacrylate, ou un copolymère contenant des résidus acrylate ou hydroxyéthylacrylate, un polyvinyle, un chitosan, ou un polysaccharide.

Ces polymères présentent préférentiellement une qualité pharmaceutique telle que définie dans la Pharmacopée Européenne ou la Pharmacopée des Etats-Unis.

De manière préférentielle, le polymère cellulosique est une hydroxypropylméthyl cellulose (HPMC), une carboxyméthylcellulose, une méthylhydroxypropylcellulose, ou encore une hydroxyéthylcellulose.

Avantageusement la couche de polymères présente une épaisseur comprise entre 1 µm et 100 µm et préférentiellement entre 2 et 10µm. Cette couche adhère sur le support par l'intermédiaire de liaisons hydrogène.

Le support, recouvert par cette couche peut être un métal, tel que de l'acier ou du titane, une céramique, ou un matériau polymérique, tel que des caoutchoucs, du polyéthylène, du polysiloxane, du polyamide, du polyéther, en particulier de marque Pebax (Atochem), des polyesters, ou du polychlorure de vinyle.

De manière générale, un tel support peut être tout matériau utilisé pour la fabrication d'instruments à usage médical.

En fonction de sa composition, le support peut être recouvert par une couche, dite intermédiaire, le séparant de la couche de polymère hydrosoluble mentionnée ci-dessus. Une telle couche a pour fonction de renforcer l'adhérence de la couche de polymère sur le support. Elle peut être constituée de polyéthylènevinylacétate (EVA).

Ces matériaux peuvent présenter, outre leurs propriétés tribologiques, des activités spécifiques, en fonction du type de polymère hydrosoluble les recouvrant. Ainsi, le chitosan est connu pour présenter de nombreuses propriétés, telles que des propriétés hémostatiques.

Les matériaux selon la présente invention peuvent être obtenus par un procédé comprenant les étapes suivantes:
- traitement de la surface du support dépourvue de polymère, par un plasma, ou par décharge couronne,
- trempage du support ainsi traité dans une solution aqueuse des polymères hydrosolubles, dont on souhaite le recouvrir, et
- séchage.

De manière préférentielle, le support est traité par un plasma radio-fréquence d'argon. Il peut être traité à une puissance d'émission du réacteur à plasma comprise entre 3 et 10 watts par litre de capacité de réacteur, durant entre environ 1 et 20 minutes. Le traitement peut aussi être effectué par un plasma micro-ondes, à la même puissance, mais durant 5 secondes à 20 minutes.

Le traitement par plasma est effectué sous vide.

Le traitement peut aussi être effectué par décharge couronne. La tension de traitement est avantageusement prise entre 50 et 500 volts, l'intensité étant variable selon le dispositif de traitement et les supports traités. La durée du traitement est de l'ordre de quelques dizièmes de seconde, préférentiellement compris entre 0,1 et 1 seconde. En cas de traitement en continu, la durée d'exposition est telle que le matériau à traiter passe à travers le dispositif de traitement à une vitesse de quelques centimères à plusieurs décimètres par seconde.

En outre les matériaux peuvent être traités à plusieurs reprises afin d'augmenter l'efficacité du traitement.

Le traitement par décharge couronne peut être effectué à l'aide de dispositifs à électrodes parallèles en vis-à-vis, à électrodes parallèles côte à côte (arc électrode d'environ 5 mm de hauteur), ou à arc souflé (électrodes parallèles côte à côte avec courant gazeux entre, créant ainsi un arc électrique d'environ 10 cm de hauteur).

Les matériaux ayant subi ces traitements peuvent être rincés, avant d'être séchés.

Le traitement par plasma ou par décharge couronne a pour objet d'améliorer la mouillabilité de la surface du matériau. Il peut être remplacé par tout autre traitement aboutissant à ce résultat.

La surface du matériau peut en outre devoir être oxydée, afin de faciliter l'adhérence de la couche de polymère hydrosoluble. Cette étape d'oxydation peut être préalable au traitement par plasma ou décharge de couronne. Elle peut aussi venir s'intercaler entre cette étape et l'étape de trempage.

L'utilisation d'un polymère hydrosoluble est particulièrement avantageuse en ce qu'elle évite l'utilisation de solvants organiques, toxiques, pour dissoudre le polymère lors du trempage du support.

En outre, les propriétés du polymère ne sont pas modifiées lors de son recouvrement du support. Il conserve notamment ses qualités, ou grade, pharmaceutiques.

La mise en oeuvre des techniques en phase gazeuse d'activation des surfaces (décharge couronne, plasma) présente l'avantage de n'induire aucune pollution, et donc d'éviter tout problème de toxicité.

En outre le traitement multidirectionnel par plasma s'applique parfaitement à des objets présentant des géométries complexes.

L'utilisation d'un plasma, ou de décharge couronne, permet de traiter l'intérieur de tubulures de faibles diamètres. Il est ensuite possible, par trempage, et si nécessaire circulation d'une solution du ou des polymères hydrophiles choisis, de déposer de façon uniforme un film homogène, aussi bien à l'intérieur qu'à l'extérieur des tubulures.

La présente invention a en outre pour objet des instruments à usage en médecine humaine ou vétérinaire constitués d'un matériau tel que décrit ci-dessus. Ces instruments peuvent être recouverts par la couche de polymère hydrosoluble décrite ci-dessus, soit sur l'ensemble de leur surface, ou soit seulement sur une partie de leurs surfaces. Ainsi, des tubulures peuvent être recouvertes totalement, ou seule leur surface interne peut être recouverte, ou encore seule leur surface externe peut être recouverte, en fonction de l'utilisation finale.

A titre non limitatif, de tels instruments peuvent être des cathéters, tels que des cathéters stomacaux, des tubes de nourrissage, des tubes d'approvisionnement en oxygène, des canules, des tubes pour trachéotomie, des cathéters pour l'aspiration intratrachéale, des cathéters destinés à être insérés dans la bouche ou le nez, des cathéters urétraux, ou rectaux, ou d'autres cathéters destinés à être insérés dans diverses cavités ou tissus corporels, des aiguilles, ainsi que des guides pour de tels cathéters. Ils peuvent être encore des endoscopes.

De tels instruments peuvent aussi être des accessoires médicaux tels que des seringues, du matériel d'implantation de prothèses ou encore du matériel ancillaire médical.

La présente invention est illustrée sans être aucunement limitée par les exemples qui suivent.

### EXEMPLE 1:

### Fabrication de tubulures constitués de supports de nature polymérique recouverts de polymères hydrosolubles.

### 1. Traitement des tubulures

Les tubulures testées sont constituées des supports suivants:
- polyamide pur (PA) commercialisé par ATOCHEM
- polyamide chargé (PA chargé) commercialisé par ATOCHEM
- polyuréthane (PU) commercialisé par BAYER (pelletane)
- caoutchouc silicone grade médical (commercialisé par DOW CORNING)
- chlorure de polyvinyle (PVC).

Ces supports ont été traités par un plasma radio-fréquence (13,56 Mhz) dans un réacteur sous vide de 20 l avec de l'argon, à 100 watts pendant cinq minutes.

### 2. Recouvrement par la couche de polymère hydrosoluble.

Des solutions aqueuses ont été préparées avec les polymères hydrosolubles suivants:
- hydroxypropylméthylcellulose (HPMC) de grade E4M, à 0,5%, commercialisé par DOW CHEMICAL,
- polyacrylamide à 2%, commercialisé par PROLABO ou SIGMA,
- polyéthylèneglycol (Lutrol 4000) à 2%, commercialisé par BASF,
- polyvinylpyrrolidone à 2% (PVP), commercialisé par BASF (KOLLIDON).

Ces polymères sont dissous à température ambiante.

Aussitôt après le traitement en surface, les tubulures sont trempées dans la solution aqueuse de polymère pendant quelques minutes, puis sont retirées, égouttées et séchées, soit à l'air soit en étuve.

### 3. Evaluation du glissement.

En l'absence de tests normalisés de mesure du coefficient de glissement sur des tubulures, le glissement a été évalué par le passage des doigts humides d'un expérimentateur sur la surface des tubulures.

L'angle de contact (θ) avec l'eau a en outre été mesuré. Les résultats sont exprimés dans le tableau I ci-après.

On constate que les tubulures recouvertes d'hydroxypropylméthylcellulose présentent en milieu humide le meilleur caractère glissant. Le caractère glissant de ce matériau est très nettement supérieur à celui de produits connus pour leurs propriétés tribologiques, tels que le polyacrylamide.

### EXEMPLE 2:

### Fabrication de tubulures recouvertes d'une couche de polymère cellulosique hydrosoluble.

Les conditions de fabrication des matériaux sont identiques à celles de l'exemple 1.

Les polymères utilisés pour recouvrir les matériaux sont l'hydroxypropylméthylcellulose (HPMC) présentant les grades K4M, K15M, Culminal ou E4M (commercialisés par HERCULES/AQUALON et DOW CHEMICAL). Ces grades sont de qualité pharmaceutique.

Les tubulures ainsi traitées ont été séchées à l'air ventilé, après trempage dans les solutions de polymère.

Le glissement a été évalué de diverses manières:
- par passage des doigts humides sur la surface du matériau après séchage.
- après trempage des tubulures recouvertes, 1 heure à 37°C dans du liquide physiologique (NaCI 0,9%), suivi d'un séchage, et
- après lavage dans un courant d'eau.

Les résultats sont exprimés dans les tableaux Il à V, respectivement pour les polymères E4M, K15M, Culminal et K4M.

Ces résultats montrent clairement que le caractère glissant du revêtement est conservé, même après trempage dans une solution de liquide physiologique durant 1 heure à 37°C, et après lavage sous un courant d'eau.

### EXEMPLE 3:

### Traitement de joints de piston de seringue selon le procédé de l'invention.

Pour les joints de caoutchouc de seringue, le problème technique à résoudre consiste en l'obtention d'un remplissage et d'un vidage facilité du fait d'un frottement réduit entre le joint en caoutchouc du piston et l'intérieur du corps de seringue.

Les données expérimentales ci-dessous montrent que ce problème a été résolu grâce au traitement des joints de caoutchouc des seringues par le procédé de l'invention.
1. Traitement des joints en caoutchouc:
   Les conditions de fabrication sont identiques à celles de l'exemple 1.
   a) Traitement par plasma (RF, argon). Le traitement par plasma radio-fréquence (RF) est réalisé à la longueur d'onde de 13,56 Mhz pendant 10 minutes.
   b) Trempage dans une solution aqueuse de polymère hydrophile de polyvinylpyrrolidone (PVP) pendant quelques minutes (1-10 minutes). La durée du trempage n'est pas déterminante et, dans certains cas, un trempage dans la solution aqueuse de polymère pendant quelques secondes peut être suffisant pour assurer un recouvrement satisfaisant du support par ce polymère.
   c) Séchage à 60°C pendant 1 heure dans une étuve ventillée à courant d'air chaud.
2. Mesures comparatives des forces de frottement avec des caoutchoucs de seringue non traités, ou traités selon le procédé de l'invention.
   Des mesures de force ont été déterminées dans les conditions suivantes:
   - Seringue Volume: 50 ml
      Diamètre: 30 mm
      Nature du matériau: polypropylène
   - Le corps de la seringue est rempli d'eau.
   - Les mesures sont effectuées sans aiguille.
   - On utilise un dynamomètre qui est appuyé sur le piston et la force nécessaire au coulissement du piston dans la seringue est lue directement.

### Résultats:

| | |
|---|---|
| Seringue non traitée, à sec | F ≈ 0,20 Newton |
| Seringue non traitée, avec de l'eau | F ≈ 0,15 Newton |
| Seringue traitée PVP, avec eau | |
| 1er essai | F ≈ 0,095 Newton |
| 2ème essai | F ≈ 0,095 Newton |
| 3ème essai | F ≈ 0,105 Newton |

Les résultats ci-dessus montrent que l'utilisation de seringues munies d'un piston dont le joint de caoutchouc a été traité selon le procédé de l'invention, nécessite une force d'appui diminuée de plus de 36% par rapport à la force d'appui nécessaire au coulissement des pistons de seringue non traités.

**TABLEAU I**

| Support (traité plasma) | HPMC | | Polyacrylamide | | Lutrol | | PVP | |
|---|---|---|---|---|---|---|---|---|
| | Glissement | θ(°) | Glissement | θ(°) | Glissement | θ(°) | Glissement | θ(°) |
| Silicone | ++ | 43 | - | 27 | - | 16 | + | 32 |
| PA pur | ++ | 48 | + | 16 | - | 16 | + | 26 |
| PA chargé | ++ | 49 | + | 15 | - | 17 | - | 24 |

**TABLEAU Il**

| Support | | Dépôt E4M | | | | |
|---|---|---|---|---|---|---|
| Matériau | Plasma | Dépôt | | + 1h/37°C / sérum phys. | | + lavage |
| | | Epaisseur (microns) | Glissant | Epaisseur (microns) | Glissant | |
| PA chargé | Non | | ++ | | + | |
| | Oui | | ++ | | + | |
| PA | Non | 5 | ++ | 1 | + | 0 |
| | Oui | 4 | ++ | 1 | ++ | + |
| Silicone | Non | | 0 | | | |
| | Oui | | ++ | | | |
| PU | Non | | + | | + | |
| | Oui | | ++ | | + | |
| PVC | Non | | + | | | |
| | Oui | | ++ | | | |

**TABLEAU III**

| Support | | Dépôt E4M | | | | |
|---|---|---|---|---|---|---|
| Matériau | Plasma | Dépôt | | + 1h/37°C / sérum phys. | | + lavage |
| | | Epaisseur (microns) | Glissant | Epaisseur (microns) | Glissant | |
| PA chargé | Non | | ++ | | + | 0 |
| | Oui | | ++ | | ++ | 0 |
| PA | Non | 4 | ++ (poisseux) | 1 | + | 0 |
| | Oui | 4 | ++ (poisseux) | 1 | ++ | + |
| PU | Non Oui | | ++ ++ | | + ++ | |

**Tableau IV**

| Support | | Dépôt Culminal | | | | |
|---|---|---|---|---|---|---|
| Matériau | Plasma | Dépôt | | + 1h/37°C / sérum phys. | | + lavage |
| | | Epaisseur (microns) | Glissant | Epaisseur (microns) | Glissant | |
| PA chargé | Non | | ++ | | + | 0 |
| | Oui | | ++ | | + | 0 |
| PA | Non | 5 | ++ (poisseux) | 1 | + | 0 |
| | Oui | 4 | ++ (poisseux) | 1 | ++ | + |
| PU | Non | | ++ | | + | |
| | Oui | | ++ | | ++ | |

**Tableau V**

| Support | | Dépôt K4M | | | | |
|---|---|---|---|---|---|---|
| Matériau | Plasma | Dépôt | | + 1h/37°C / sérum phys. | | + lavage |
| | | Epaisseur (microns) | Glissant | Epaisseur (microns) | Glissant | |
| PA chargé | Non | | ++ | | + | 0 |
| | Oui | | ++ | | + | 0 |
| PA | Non | 5 | ++ (poisseux) | 1 | + | 0 |
| | Oui | 4 | ++ (poisseux) | 1 | + | 0 |
| PU | Non | | ++ | | + | |
| | Oui | | ++ | | ++ | |

## Revendications

1. Matériau à faible coefficient de glissement caractérisé en ce qu'il est constitué d'un support, modifié en surface par création de sites polaires, recouvert par une couche d'au moins un polymère hydrosoluble et biocompatible.

2. Matériau selon la revendication 1 caractérisé en ce qu'il est modifié en surface sur une épaisseur comprise entre 10 et 100 nm.

3. Matériau selon l'une des revendications 1 et 2 caractérisé en ce que la couche de polymère hydrosoluble présente une épaisseur comprise entre 1 et 100 µm.

4. Matériau selon la revendication 1 caractérisé en ce que ledit polymère hydrosoluble est un dérivé cellulosique, une polyvinylpyrrolidone, ou un copolymère de polyvinylpyrrolidone, un polypropylèneglycol, un polyacrylamide, un polyacrylate, ou un copolymère contenant des résidus acrylate ou hydroxyéthylacrylate, un polyvinyle, un chitosan, ou un polysaccharide.

5. Matériau selon la revendication 2 caractérisé en ce que ledit polymère hydrosoluble est une hydroxypropylméthyl cellulose (HPMC), une carboxyméthylcellulose, une méthylhydroxypropylcellulose, ou encore une hydroxyéthylcellulose.

6. Matériau selon l'une des revendications 1 à 3 caractérisé en ce que le support est un matériau polymérique, un métal ou une céramique.

7. Matériau selon l'une des revendications 1 à 6 caractérisé en ce qu'il comporte une couche intermédiaire, située entre le support et la couche de polymère hydrosoluble.

8. Procédé de fabrication d'un matériau selon l'une des revendications 1 à 7 comprenant les étapes suivantes:
- traitement du support par un plasma ou par décharge couronne,
- trempage du support ainsi traité dans une solution aqueuse de polymère hydrosoluble, et
- séchage.

9. Procédé selon la revendication 8 caractérisé en ce que le support est traité par un plasma d'argon.

10. Procédé selon l'une des revendications 8 et 9 caractérisé en ce que le support est traité par un plasma radio-fréquence à une puissance d'émission du réacteur à plasma comprise entre 3 et 10 watts par litre de capacité de réacteur, durant 1 à 20 minutes.

11. Procédé selon l'une des revendications 8 et 9 caractérisé en ce que le support est traité par un plasma de type micro-ondes à une puissance d'émission du réacteur à plasma comprise entre 3 et 10 watts par litre de capacité de réacteur durant 5 secondes à 20 minutes.

12. Procédé selon l'une des revendications 8 à 11 caractérisé en ce que la surface du support est oxydée, préalablement au traitement par plasma ou par décharge couronne.

13. Instrument à usage médical caractérisé en ce qu'il est constitué d'un matériau selon l'une des revendications 1 à 7, ou obtenu par le procédé selon l'une des revendications 8 à 12.

14. Instrument selon la revendication 13 caractérisé en ce qu'il est une tubulure.

15. Instrument selon la revendication 13, caractérisé en ce qu'il est un joint de caoutchouc du piston d'une seringue.
